# FASCICULE DE BREVET EUROPEEN

(11) **EP 0 795 763 B1**
(45) Date de publication et mention de la délivrance du brevet: **24.07.2002**
(21) Numéro de dépôt: 97400452.5
(22) Date de dépôt: 27.02.1997
(51) Int. Cl.: G01T 1/00, G01T 1/29

(54) **Dispositif d'imagerie multicoupes**
Vorrichtung zur Transversabschichtbildern
Cross-sectional imaging device

(30) Priorité: 29.02.1996 FR 9602548
(43) Date de publication de la demande: 17.09.1997
(73) Titulaire: COMMISSARIAT A L'ENERGIE ATOMIQUE, 75015 Paris (FR)
(72) Inventeur: Glasser, Francis, 38320 Eybens (FR); Peyret, Olivier, 38120 Le Fontanil (FR)
(74) Mandataire: Dubois-Chabert, Guy

(56) Documents cités:
- EP-A- 0 571 135
- US-A- 4 963 746
- US-A- 5 245 191
- US-A- 5 291 402

## Description

### Domaine technique

La présente invention concerne un dispositif d'imagerie multicoupes. Elle s'applique, en particulier, aux dispositifs d'imagerie en transmission X, en transmission γ, ou en émission γ.

### Etat de la technique antérieure

Les tomographes X médicaux de l'art antérieur utilisent comme détecteurs soit des détecteurs à gaz, soit des scintillateurs associés à des photodiodes. Mais de tels détecteurs ne réalisent que l'acquisition d'une seule coupe à chaque rotation de l'ensemble source-détecteur. Les cartographies en volume sont réalisées par l'acquisition de plusieurs coupes avec, à chaque coupe, un déplacement du patient perpendiculairement au faisceau de rayons X. A chaque coupe, l'ensemble détecteur-source de rayonnement effectue une rotation complète autour du patient.

Il existe également des machines qui enchaînent les coupes en déplaçant de façon continue le patient. Le logiciel de reconstruction prend alors en compte cette acquisition de type hélicoïdal.

Une autre solution consiste à utiliser un détecteur bidimensionnel de type intensificateur d'image radiologique, et à acquérir par une rotation complète un ensemble d'images bidimensionnelles. L'image du volume est reconstruite directement à partir de ces acquisitions, comme dans le tomographe 3D (3 dimensions) décrit dans un article référencé [1] en fin de description.

Un document référencé [2] décrit un tomographe à rotation continue enchaînant les acquisitions.

Plusieurs documents décrivent l'utilisation de détecteurs semi-conducteurs en tomographie :

Un document référencé [3] décrit un appareil de détection de radiations sous forme d'impulsions de durée prédéterminée, comprenant un détecteur à semi-conducteur, qui crée une charge électronique sous l'action du rayonnement incident, le détecteur étant destiné à recevoir une partie au moins des impulsions des radiations. Un dispositif de traitement de signaux est couplé en courant alternatif au détecteur à semi-conducteur et destiné à former un signal électrique de sortie représentant avec précision les radiations incidentes. Le dispositif de traitement de signaux a un filtre ne permettant la contribution au signal électrique de sortie que des composantes dont la fréquence se trouve dans une bande prédéterminée de fréquences, cette bande étant déterminée en fonction de la durée des impulsions incidentes si bien que le signal électrique de sortie ne présente pas de distorsions dues aux variations des caractéristiques électriques internes du détecteur à semi-conducteur. Mais l'appareil tel que décrit dans ce document référencé [3] ne donne pas de bons résultats et n'est pas utilisé depuis 1979 : il y a en effet un problème de traînée de la réponse du détecteur soumis au rayonnement X.

Un document référencé [4] décrit un détecteur semi-conducteur connecté à un circuit de lecture par bille d'indium, pour lequel une application est possible en tomographie.

Les possibilités d'évolution de ces détecteurs sont les suivantes :
- Pour les détecteurs à gaz : il y a eu des tentatives pour faire évoluer les détecteurs à gaz afin de pouvoir acquérir plusieurs coupes en même temps. Mais une solution de ce type n'a toujours pas été adoptée à ce jour. De plus, le nombre de coupes possibles dans une configuration de ce type reste très limité (inférieur à 10).
- Pour les détecteurs scintillateurs associés à des photodiodes : les scintillateurs convertissent le rayonnement X en lumière visible. Cette lumière est mesurée par des photodiodes qui sont déportées sur le côté pour ne pas être détériorées par le faisceau X. Une telle structure pourrait évoluer afin de réaliser deux ou trois coupes en même temps, mais guère plus. Pour effectuer réellement un grand nombre de coupes avec ce type de détecteur, il faudrait pouvoir accoupler directement le scintillateur aux photodiodes par l'intermédiaire de fibres optiques. Mais, dans ce cas, les photodiodes risquent de se détériorer très rapidement.

Ainsi, dans le domaine de l'imagerie médicale, les scanners utilisent des détecteurs à gaz ou à scintillateurs qui font l'acquisition d'une coupe, exceptionnellement deux coupes, à chaque rotation de l'ensemble source-détecteur. Pour acquérir une information en volume, il est nécessaire de réaliser plusieurs coupes dont la hauteur est fixée pour un examen donné.

De plus, l'utilisation de détecteurs à gaz ou de scintillateurs nécessite des moyens de conversion pour récupérer une information électrique, et les dispositifs de détection qui les utilisent sont de ce fait encombrants.

Pour cette raison aussi, les dispositifs de l'art antérieur ne permettent pas d'aligner un nombre de barrettes de détecteurs supérieur à deux.

D'autres dispositifs analogues sont divulgués dans US-A-4 963 746, US-A-5 245 191 et US-A-5 291 402.

L'utilisation de détecteurs semi-conducteurs pour la détection de rayonnement ionisant, dans le cadre de l'imagerie médicale ou du contrôle non destructif, permet d'envisager de nouvelles fonctionnalités sur les dispositifs de détection, comme faire l'acquisition simultanée de plusieurs coupes.

L'invention a pour objet d'utiliser un ensemble de tels détecteurs pour réaliser un dispositif d'imagerie multicoupes.

### Exposé de l'invention

La présente invention concerne un dispositif d'imagerie multicoupes comprenant une source de rayonnement ionisant possédant un foyer, et un ensemble de détecteurs semi-conducteurs bidimensionnels apte à recevoir ledit rayonnement après traversée d'un corps, caractérisé en ce que ces détecteurs semi-conducteurs bidimensionnels sont plans, associés les uns aux autres de façon à former un ensemble continu, de façon à ce que la perpendiculaire à chacun d'entre eux passe par le foyer de la source, et formés de détecteurs élémentaires semi-conducteurs accolés les uns aux autres afin de réaliser plusieurs couronnes de détection, apte à tourner autour dudit corps afin de permettre l'acquisition simultanée de plusieurs coupes d'imagerie, et en ce que chaque détecteur élémentaire semi-conducteur est muni d'un contact bloquant qui permet de limiter le problème de traînée de la réponse du détecteur soumis au rayonnement.

L'invention s'applique en particulier aux dispositifs d'imagerie en transmission γ et X et en émission γ.

Avantageusement, chaque détecteur bidimensionnel est réalisé à partir de détecteurs élémentaires semi-conducteurs de haute résistivité.

Avantageusement, chaque détecteur semi-conducteur est constitué d'un matériau pris dans les familles de semi-conducteurs de type IV (SiGe), II-VI (ZnS), III-V (GaAs, InP), II-VII (HgI2),par exemple :

| | | | |
|---|---|---|---|
| Cd X Te | avec par exemple | X = Zn | ou rien |
| As X Ga | " | X = Al | ou rien |
| Biₓ X Oy | " | X = Ge, Si | x=12, y=1 |
| Pb X O | " | X = Ti | ou rien |
| XSe | " | X = Cd | ou rien |

Avantageusement, le dispositif de l'invention comporte un circuit électronique de mesure qui est déporté du faisceau incident par l'intermédiaire d'un support de connexion. Les détecteurs et le circuit de lecture sont connectés à ce support par des billes métalliques par exemple à base d'indium.

Avantageusement, une électronique de lecture est connectée aux détecteurs semi-conducteurs par l'intermédiaire d'un support de connexion, et cette électronique est protégée du rayonnement par un blindage en matériau absorbant.

Avantageusement, chaque détecteur semi-conducteur est composé d'une plaque de semi-conducteur ayant sur la face destinée à être irradiée une électrode homogène et sur l'autre face en regard une multitude de petites électrodes indépendantes. Chaque petite électrode délimitant la taille d'un pixel détecteur, elle est connectée sur une bande métallique d'un support d'interconnexion, par exemple par la technique des billes métalliques, par exemple à base d'indium ou en caoutchouc conducteur. Cette bande métallique est connectée à l'électronique de lecture déportée sur le côté par la même technique.

L'ensemble examiné peut être un objet ou un sujet (patient).

L'invention concerne également un procédé d'imagerie de mise en oeuvre de ce dispositif, caractérisé en ce que, à partir d'une seule dose de rayonnement ionisant permettant de réaliser une acquisition, la hauteur de coupe peut être choisie après l'étape d'acquisition, au moment de la reconstruction d'images, en sommant la contribution d'un nombre choisi de couronnes, permettant de sélectionner au moins un volume d'examen et/ou de le faire varier.

Avantageusement, une première coupe est donnée par la reconstruction d'images de la réponse des pixels de la première colonne de tous les détecteurs plans. On acquiert en même temps autant de coupes qu'il y a de colonnes, la hauteur de coupe étant définie par la dimension du pixel élémentaire. Elle peut être augmentée pendant l'acquisition en sommant la réponse de plusieurs pixels sur une ligne hauteur de coupe.

Ainsi, selon l'invention, les détecteurs associés entre eux permettent d'acquérir en même temps plusieurs coupes d'imagerie. Ils permettent de plus une économie sur l'utilisation du tube à rayonnement, ce qui permet d'augmenter sa durée de vie.

De plus l'invention permet d'obtenir des mesures avec une résolution fine due au faible espacement entre les détecteurs, dans toutes les directions, de l'ordre de 0,1 mm ; alors que dans les dispositifs de l'art antérieur, le patient se déplaçant par rapport à la couronne de détection, la résolution dans le sens de déplacement du patient est de l'ordre du mm.

En outre, l'emploi de détecteurs semi-conducteurs permet en outre de déporter l'électronique de proximité et de la protéger du rayonnement incident.

Le dispositif de l'invention peut être utilisé en tomographie médicale, mais aussi pour réaliser un contrôle non destructif en tomographie industrielle. L'intérêt essentiel est, alors, qu'il permet de réaliser un gain en temps pour contrôler un volume donné et de détecter une zone en défaut, par exemple en tomographie de propulseur de fusée et tomographie de céramique.

### Brève description des dessins

- Les figures 1 et 2 illustrent schématiquement le dispositif de l'invention ;
- la figure 3 illustre le schéma de principe de l'interconnexion d'un détecteur avec une électronique de mesure dans le dispositif de l'invention ;
- la figure 4 illustre le schéma de principe d'un détecteur semi-conducteur dans le dispositif de l'invention.

### Exposé détaillé de modes de réalisation

L'invention concerne un dispositif d'imagerie multicoupes, qui peut être notamment un dispositif d'imagerie en transmission X, en transmission γ, ou en émission γ.

Pour simplifier la description qui va suivre, l'exemple considéré ci-après sera celui de la transmission X (tomographie X).

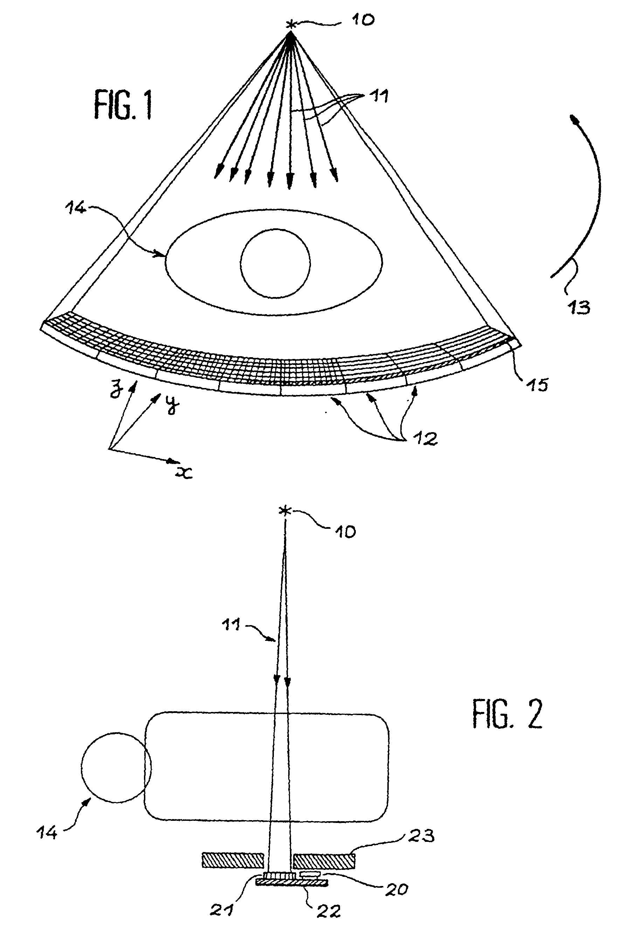

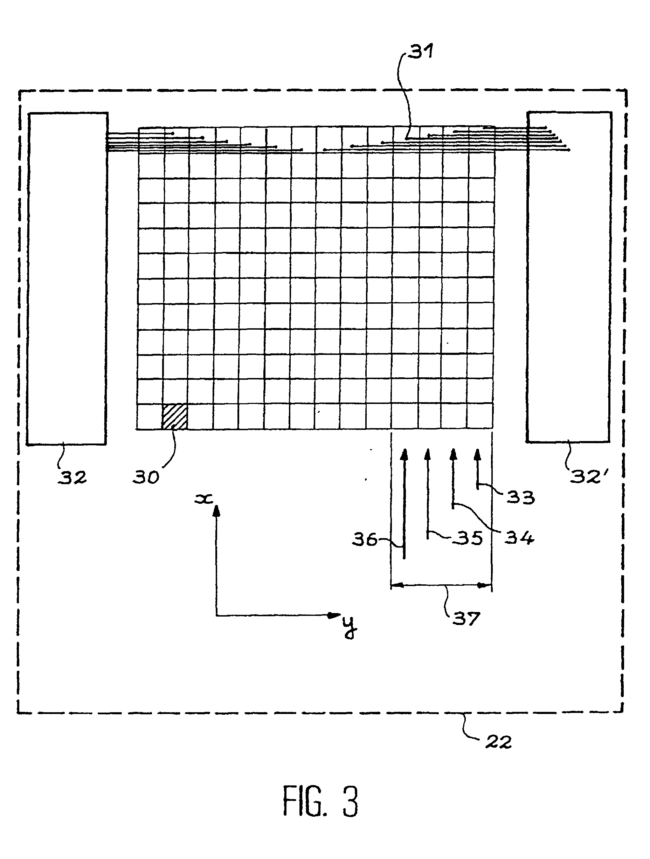

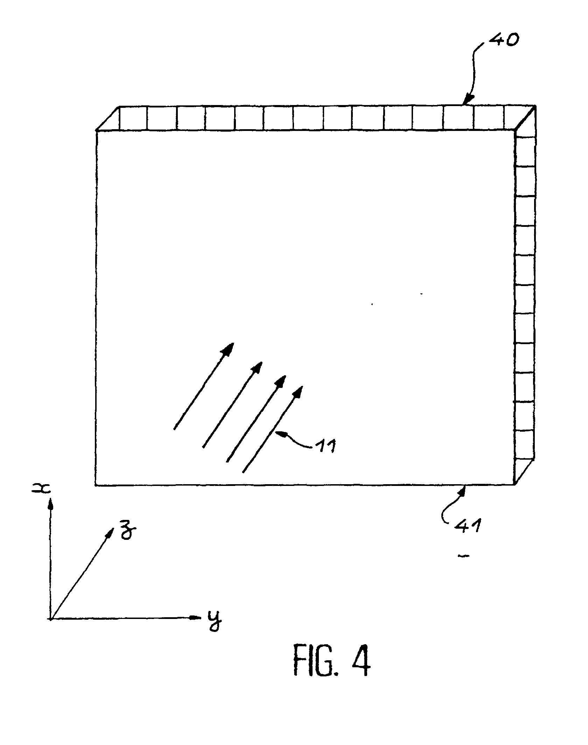

Comme représenté sur la figure 1, le dispositif tomographe, selon l'invention, est composé d'une source 10 de rayonnement X 11 et d'un ensemble de détecteurs semi-conducteurs 12 qui tournent 13 autour d'un corps ou ensemble 14, qui peut être un objet ou un sujet (sur les figures 3 et 4 l'ensemble 14 représenté est un sujet (patient)). La couronne de détection ainsi formée est elle-même composée de plusieurs détecteurs plans accolés les uns aux autres de manière à former un ensemble continu. Les détecteurs plans sont disposés de façon à ce que la perpendiculaire à chaque plan (par exemple l'axe z) passe par le foyer de la source de rayons X. La première coupe est référencée 15.

L'invention concerne ainsi un tomographe apte à acquérir des mesures suivant plusieurs coupes (correspondant avantageusement au nombre de couronnes), qui comprend un ensemble de détecteurs bidimensionnels (2D), animé d'un mouvement de rotation autour de l'ensemble 14. Chaque détecteur bidimensionnel est réalisé par exemple à partir de détecteurs élémentaires semi-conducteurs de haute résistivité, accolés les uns aux autres de façon à former deux couronnes de détection juxtaposées. Avantageusement, chaque détecteur élémentaire est muni d'un contact bloquant. Ce contact bloquant est connecté à une bille métallique par exemple à base d'indium, elle-même connectée à un circuit de lecture.

Chaque détecteur semi-conducteur peut être constitué d'un matériau pris dans les familles de semi-conducteurs de type IV (SiGe), II-VI (ZnS), III-V (GaAs, InP), II-VII (HgI2),par exemple :

| | | | |
|---|---|---|---|
| Cd X Te | avec par exemple | X = Zn | ou rien |
| As X Ga | " | X = Al | ou rien |
| Biₓ X Oy | " | X = Ge, Si | x=12, y=1 |
| Pb X O | " | X = Ti | ou rien |
| XSe | " | X = Cd | ou rien |

L'invention concerne également un procédé tomographique utilisant ce dispositif qui, à partir d'une seule dose de rayonnement X, permet de réaliser une acquisition. La hauteur de coupe peut être choisie après l'étape d'acquisition, au moment de la reconstruction d'images, en sommant la contribution d'un nombre choisi de couronnes, permettant de sélectionner au moins un volume d'examen et/ou de le faire varier.

La figure 2 montre le même dispositif en coupe. L'électronique de lecture 20 est connectée au détecteur semi-conducteur 21 par l'intermédiaire d'un support de connexion 22. Elle est protégée du rayonnement X par un blindage 23 en matériau absorbant.

Comme représenté sur la figure 3, chaque détecteur élémentaire est composé d'une plaque de semi-conducteur ayant sur la face destinée à être irradiée une électrode homogène et sur l'autre face en regard une multitude de petites électrodes indépendantes. Chaque petite électrode délimite la taille d'un pixel détecteur 30. Elle est connectée à une bande métallique d'un support d'interconnexion par la technique des billes métalliques par exemple à base d'indium 31, ou en caoutchouc conducteur. Cette bande métallique est connectée à une électronique de lecture 32 (32') déportée sur le côté par la même technique.

Le circuit de lecture peut être réalisé en technologie durcie au rayonnement, mais son coût est alors élevé. Ce type de circuit pourra être préféré dans le cas où le dispositif comporte un grand nombre de couronnes de détection.

Avantageusement, le circuit de lecture est déporté de l'ensemble de détecteurs via des pistes (une piste par détecteur élémentaire) et est muni d'un écran absorbant les rayonnements X de façon à protéger le circuit de lecture. Ce mode permet de faire tenir l'ensemble des connexions sur la largeur d'un pixel.

L'intérêt de la solution semi-conducteur est de convertir directement le rayonnement X en charges électriques. Les électrodes de collection du détecteur peuvent être connectées à un support par la technique de billes métalliques par exemple à base d'indium ("flip-chip"). Le circuit électronique de mesure est, lui aussi, connecté à ce support par la même technique mais de façon déportée pour être protégé du faisceau X incident par un blindage métallique. Le support réalise alors l'interconnexion entre le détecteur et le circuit de lecture par des bandes métalliques, comme représenté sur la figure 3.

En accolant ce type de détecteurs plans en couronne, on peut ainsi réaliser un grand nombre de coupes en même temps. Chaque coupe est définie par un pixel sur l'axe Y. On peut modifier la hauteur de coupe pendant l'acquisition en sommant les signaux d'un certain nombre de pixels de l'axe Y.

La première coupe 33 est donnée par la reconstruction d'image de la réponse de pixels de la première colonne de tous les détecteurs plans. On acquiert en même temps autant de coupes qu'il y a de colonnes. La hauteur de coupe est définie par la dimension du pixel élémentaire. Elle peut être augmentée en sommant la réponse de plusieurs pixels sur une ligne.

Sur la figure 3 sont référencées la première coupe 33, la seconde coupe 34, la troisième coupe 35 et la quatrième coupe 36. Sur cette figure est également référencée la hauteur de coupe variable 37.

Sur la figure 4, qui illustre un schéma de principe du détecteur semi-conducteur, sont référencées la face avant 40 qui est une électrode pleine couche et la face arrière 41 qui est une électrode "pixelisée".

L'invention décrit l'utilisation de détecteurs à base de semi-conducteur pour réaliser des dispositifs d'imagerie multicoupes, par exemple des tomographes, utilisation rendue possible par l'utilisation de contacts bloquants (comme par exemple l'aluminium, l'indium, l'argent). De tels contacts bloquants, qui sont stables dans le temps pour un rayonnement ionisant, par exemple un rayonnement X, permettent d'améliorer grandement la qualité de la détection.

On va considérer à présent plusieurs exemples comparatifs d'application :

Typiquement, pour localiser un objet donné, avec un dispositif comportant une barrette de détecteurs de 12 mm de large, d'un mètre de long, qui fait le tour d'un patient et un collimateur pour diminuer la largeur de coupe entre deux expériences, on a les étapes suivantes :
- on fixe une largeur de coupe, par exemple de 10 mm ;
- on fait un certain nombre de coupes, par exemple cinq, pour inspecter 50 mm de large ;
- sur une coupe apparaît une anomalie ;
- on se repositionne par rapport à l'anomalie ;
- on recommence le procédé en diminuant la largeur de coupe, par exemple 2 mm, et on refait cinq coupes.

On peut continuer mais, à chaque coupe, on cumule les doses de rayonnement au même endroit du corps (5 rem à chaque fois). De plus, ce procédé est long.

Par ailleurs, il est difficile d'aligner un grand nombre de détecteurs sur une même barrette.

Par contre, avec le dispositif de l'invention, pour un détecteur de 25 mm de large avec 1 000 pixels, la largeur de la coupe est égale au pixel : 250 µm, on a une électrode par point élémentaire et un espace mort entre deux pixels pour éviter les courts-circuits :
- on effectue une rotation ;
- avec une seule dose de rayonnement, on fait 100 coupes pour inspecter une largeur de 50 mm en obtenant une information par pixel ;
- à partir des données acquises, on peut reconstruire tout le volume sur 25 mm pour avoir une vision globale et/ou reconstruire sur des largeurs plus petites à choisir.

Autrement dit, avec le procédé de l'invention, pour inspecter un volume dont la hauteur maximum correspond à la hauteur formée par l'empilement des couronnes de détecteurs, on peut procéder de plusieurs façons :
- on peut sélectionner une couronne ou un ensemble de couronnes pour réaliser une image haute résolution de la zone choisie à l'aide d'un procédé de sommation de la réponse des couronnes, puis on reconstruit ;
- on peut partitionner ou échantillonner la zone d'examen : par exemple on a 100 coupes avec un pas de 250 µm ;
- on peut faire une reconstruction tous les millimètres avec une hauteur de coupe de 250 µm, on peut reconstruire une coupe tous les millimètres avec une résolution de 1 mm ;
- on peut faire une coupe de hauteur 10 mm.

L'invention permet d'obtenir une meilleure résolution parce que les coupes peuvent faire 250 µm de hauteur alors que les dispositifs de l'art antérieur donnent des coupes plus larges.

Un des avantages de l'invention est de régler le problème de positionnement par rapport, par exemple, à une tumeur, que présentaient les dispositifs de l'art antérieur.

A partir des mesures obtenues par le dispositif d'imagerie de l'invention, la reconstruction des images est réalisée par exemple de façon classique à partir d'une projection, en utilisant une transformée de Radon 2D ou 3D.

### REFERENCES

- [1]: "Development of a 3D CT-Scanner Using Cone Beam" de Masahiro Endo, Nozomu Kamagata, Kazumasa Sato, Yuichi Hattori, Shigeo Kobayaghi, Shin-Ichi Mizuno, Masao Jimbo et Masahiro Kusakabe (SPIE, volume 2432, pages 291 à 297)
- [2]: "New CT Scanner - Initial Clinical Experience" de C. Becker, U. Fink, M. Seemann et M. Reiser (Electromedica 63 ; 1995 ; n° 1)
- [3]: FR-A-2 432 718 ; "Appareil de détection de radiations pour tomographie" de "The Regents of the University of California", 26 mars 1979
- [4]: EP-A-0 571 135 ; "Hybridized Semiconductor Pixel Detector Arrays for Use in Digital Radiography" de Timothy Collins, Stuart Worley, Gordon Kramer et W. Douglas Wolfe ; 13 mai 1993

## Revendications

1. Dispositif d'imagerie multicoupes comprenant une source (10) de rayonnement ionisant (11) possédant un foyer, et un ensemble de détecteurs semi-conducteurs bidimensionnels (12) apte à recevoir ledit rayonnement (11) après traversée d'un corps (14), **caractérisé en ce que** ces détecteurs semi-conducteurs bidimensionnels sont plans, associés les uns aux autres de façon à former un ensemble continu, de façon à ce que la perpendiculaire à chacun d'entre eux passe par le foyer de la source, et formés de détecteurs élémentaires semi-conducteurs accolés les uns aux autres afin de réaliser plusieurs couronnes de détection, apte à tourner autour dudit corps (14) afin de permettre l'acquisition simultanée de plusieurs coupes d'imagerie, et **en ce que** chaque détecteur élémentaire semi-conducteur est muni d'un contact bloquant qui permet de limiter le problème de traînée de la réponse du détecteur soumis au rayonnement.

2. Dispositif selon la revendication 1, **caractérisé en ce que** la source de rayonnement ionisant (11) est une source de rayons X.

3. Dispositif selon la revendication 1, **caractérisé en ce que** le rayonnement ionisant (11) est une source de rayons γ.

4. Dispositif selon la revendication 1, **caractérisé en ce que** chaque détecteur bidimensionnel est réalisé à partir de détecteurs élémentaires semi-conducteurs de haute résistivité.

5. Dispositif selon la revendication 4, **caractérisé en ce que** chaque détecteur semi-conducteur est constitué d'un matériau pris dans les familles de semi-conducteurs de type IV (SiGe), II-VI (ZnS), III-V (GaAs, InP), II-VII (HgI2).

6. Dispositif selon la revendication 1, **caractérisé en ce qu'**il comporte un circuit électronique de mesure qui est déporté du faisceau incident par l'intermédiaire d'un support de connexion.

7. Dispositif selon la revendication 6, **caractérisé en ce que** les détecteurs et le circuit de lecture sont connectés à ce support par des billes métalliques par exemple à base d'indium.

8. Dispositif selon la revendication 6, **caractérisé en ce que** l'électronique de lecture (20) est connectée aux détecteurs semi-conducteurs (21) par l'intermédiaire d'un support de connexion (22) et **en ce que** cette électronique (20) est protégée du rayonnement par un blindage (23) en matériau absorbant.

9. Dispositif selon la revendication 1, **caractérisé en ce que** chaque détecteur semi-conducteur est composé d'une plaque de semi-conducteur ayant sur la face destinée à être irradiée une électrode homogène et sur l'autre face en regard une multitude de petites électrodes indépendantes, chaque petite électrode délimitant la taille d'un pixel détecteur (30).

10. Dispositif selon la revendication 9, **caractérisé en ce que** chaque petite électrode est connectée à une bande métallique d'un support d'interconnexion par la technique des billes métalliques (31), par exemple à base d'indium, ou en caoutchouc conducteur.

11. Dispositif selon la revendication 10, **caractérisé en ce que** cette bande métallique est connectée à l'électronique de lecture (32, 32') déportée sur le côté par la technique des billes d'indium.

12. Dispositif selon la revendication 1, **caractérisé en ce que** ledit corps (14) est un objet.

13. Dispositif selon la revendication 1, **caractérisé en ce que** ledit corps (14) est un patient.

14. Procédé d'imagerie de mise en oeuvre du dispositif selon l'une quelconque des revendications 1 à 13, **caractérisé en ce que** à partir d'une seule dose de rayonnement ionisant permettant de réaliser une acquisition, la hauteur de coupe peut être choisie après l'étape d'acquisition,au moment de la reconstruction d'images, en sommant la contribution d'un nombre choisi de couronnes, permettant de sélectionner au moins un volume d'examen et/ou de le faire varier.

15. Procédé selon la revendication 14, **caractérisé en ce qu'**une première coupe (33) est donnée par la reconstruction d'images de la réponse des pixels de la première colonne de tous les détecteurs plans, et **en ce qu'**on acquiert en même temps autant de coupes qu'il y a de colonnes, la hauteur de coupe étant définie par la dimension du pixel élémentaire.

16. Procédé selon la revendication 15, **caractérisé en ce que** la hauteur de coupe est augmentée pendant l'acquisition en sommant la réponse de plusieurs pixels sur une ligne.

## Patentansprüche

1. Multischnitt-Bildherstellungsvorrichtung, eine Quelle (10) ionisierender Strahlung (11) mit einem Brennpunkt und eine Anordnung von zweidimensionalen Halbleiterdetektoren (12) umfassend, die fähig sind, die genannte Strahlung (11) nach Durchquerung eines Körpers (14) zu empfangen,
**dadurch gekennzeichnet, dass** diese zweidimensionalen Halbleiterdetektoren plan sind und einander so zugeordnet sind, dass sie eine kontinuierliche Anordnung bilden, derart, dass die Senkrechte von jedem von ihnen durch den Brennpunkt der Quelle verläuft, wobei sie durch elementare Halbleiterdetektoren gebildet werden, die so aneinandergefügt sind, dass sie mehrere Detektionsringe bilden, die sich um den genannten Körper (14) drehen können, um die simultane Erfassung von mehreren Bildherstellungsschnitten zu ermöglichen,
und dadurch, dass jeder elementare Halbleiterdetektor mit einem Sperrkontakt versehen ist, der ermöglicht, das Widerstands- bzw. Unschärfeproblem bei der Reaktion des der Strahlung ausgesetzten Detektors zu begrenzen.

2. Vorrichtung nach Anspruch 1, **dadurch gekennzeichnet, dass** die Quelle der ionisierenden Strahlung (11) eine Röntgenstrahlenquelle ist.

3. Vorrichtung nach Anspruch 1, **dadurch gekennzeichnet, dass** die ionisierende Strahlung (11) eine Gammastrahlenquelle ist.

4. Vorrichtung nach Anspruch 1, **dadurch gekennzeichnet, dass** jeder zweidimensionale Detektor durch elementare Halbleiterdetektoren von hoher Resistivität gebildet wird.

5. Vorrichtung nach Anspruch 4, **dadurch gekennzeichnet, dass** jeder zweidimensionale Detektor durch ein Material gebildet wird, das ausgewählt wird aus den Gruppen der Halbleiter des Typs IV (SiGe), II-VI (ZnS), III-V (GaAs, InP), II-VII (HgI₂).

6. Vorrichtung nach Anspruch 1, **dadurch gekennzeichnet, dass** sie eine elektronische Messschaltung umfasst, die zum einfallenden Strahl mittels eines Verbindungsträgers seitlich versetzt ist.

7. Vorrichtung nach Anspruch 6, **dadurch gekennzeichnet, dass** die Detektoren und die Leseschaltung mit diesem Träger z.B. durch metallische Kugeln auf Indiumbasis verbunden sind.

8. Vorrichtung nach Anspruch 6, **dadurch gekennzeichnet, dass** die Leseelektronik (20) mit den Halbleiterdetektoren (21) durch einen Verbindungsträger (22) verbunden ist, und dadurch, dass diese Elektronik (20) durch eine Abschirmung (23) aus absorbierendem Material gegen die Strahlung geschützt ist.

9. Vorrichtung nach Anspruch 1, **dadurch gekennzeichnet, dass** jeder Halbleiterdetektor durch ein Halbleiterplättchen gebildet wird, das auf der zur Bestrahlung bestimmten Seite eine homogene Elektrode umfasst und auf der anderen, entgegengesetzten Seite eine Vielzahl kleiner unabhängiger Elektroden, wobei jede kleine Elektrode die Größe eines Detektorpixels (30) hat.

10. Vorrichtung nach Anspruch 9, **dadurch gekennzeichnet, dass** jede kleine Elektrode mit einem Metallband eines Zwischenverbindungsträgers verbunden ist, mittels der Metallkugelntechnik mit Kugeln (31) aus Metall auf der Basis von Indium oder aus leitfähigem Kautschuk.

11. Vorrichtung nach Anspruch 10, **dadurch gekennzeichnet, dass** dieses Metallband mit der seitlich versetzten Leseelektronik (32, 32') durch die Indiumkugelntechnik verbunden ist.

12. Vorrichtung nach Anspruch 1, **dadurch gekennzeichnet, dass** der genannte Körper (14) ein Gegenstand ist.

13. Vorrichtung nach Anspruch 1, **dadurch gekennzeichnet, dass** der genannte Körper (14) ein Patient ist.

14. Bildherstellungsverfahren zur Benutzung der Vorrichtung nach einem der Ansprüche 1 bis 13, **dadurch gekennzeichnet, dass** auf Grund einer einzigen Dosis ionisierender Strahlung, die die Durchführung einer Erfassung ermöglicht, die Schnitthöhe nach dem Erfassungsschritt gewählt werden kann, zum Zeitpunkt der Bildrekonstruktion, indem der Beitrag einer ausgewählten Anzahl von Detektorenringen summiert wir, was ermöglicht, wenigstens ein Prüfungsvolumen zu selektieren und/oder es zu variieren.

15. Verfahren nach Anspruch 14, **dadurch gekennzeichnet, dass** ein erster Schnitt (33) geliefert wird durch die Rekonstruktion von Abbildungen der Reaktion der Pixel der ersten Spalte aller planen Detektoren, und dadurch, dass man gleichzeitig ebenso viele Schnitte erfasst, wie es Spalten gibt, wobei die Schnitthöhe durch die Dimension des elementaren Pixels definiert wird.

16. Verfahren nach Anspruch 15, **dadurch gekennzeichnet, dass** die Schnitthöhe während der Erfassung erhöht wird, indem man die Reaktion mehrerer Pixel in einer Zeile summiert.

## Claims

1. Multisection imaging device incorporating an ionizing radiation (11) source (10) having a focus and an array (12) of semiconductor detectors able to receive said radiation (11) which, has traversed a body (14), **characterized in that** said bidimensional semiconductor detectors are planar and associated with one another so as to form a continuous array, so that the perpendicular to each of them passes through the focus of the source and which are formed by elementary semiconductor detectors which are joined to one another in order to obtain several detection rings, able to rotate about said body (14) in order to permit the simultaneous acquisition of several imaging sections, and **in that** each elementary semiconductor detector is provided with a blocking contact making it possible to limit the response lag problem of the detector exposed to radiation.

2. Device according to claim 1, **characterized in that** the ionizing radiation (11) source is a X-ray source.

3. Device according to claim 1, **characterized in that** the ionizing radiation (11) source is a γ-ray source.

4. Device according to claim 1, **characterized in that** each bidimensional detector is produced on the basis of high resistivity, elementary semiconductor detectors.

5. Device according to claim 4, **characterized in that** each semiconductor detector is made from a material taken from within the semiconductor families of type IV (SiGe), II-VI (ZnS), III-V (GaAs, InP), II-VII (HgI2).

6. Device according to claim 1, **characterized in that** it has an electronic measuring circuit displaced with respect to the incident beam by means of a connection support.

7. Device according to claim 6, **characterized in that** the detectors and the reading circuit are connected to said support by metal balls, e.g. based on indium.

8. Device according to claim 6, **characterized in that** the reading electronics (20) is connected to the semiconductor detectors (21) by means of a connection support (22) and **in that** said electronics (20) is protected from the radiation by an absorbing material shield (23).

9. Device according to claim 1, **characterized in that** each semiconductor detector is constituted by a semiconductor plate or chip having on the face to be irradiated a homogeneous electrode and on the other facing face a plurality of small independent electrodes, each small electrode defining the size of a detector pixel (30).

10. Device according to claim 9, **characterized in that** each small electrode is connected to a metal strip of an interconnection support by the metal ball method (31), e.g. based on indium, or of conductive rubber.

11. Device according to claim 10, **characterized in that** said metal strip is connected to the reading electronics (32, 32') offset to the side by the indium ball method.

12. Device according to claim 1, **characterized in that** the said body (14) is an object.

13. Device according to claim 1, **characterized in that** said body (14) is a patient.

14. Imaging process performed by the device according to any one of the claims 1 to 13, **characterized in that**, on the basis of a single ionizing radiation dose making it possible to carry out an acquisition, the section height can be chosen following the acquisition stage, at the time of reconstructing images, by summating the contribution of a chosen number of rings, permitting the selection of at least one examination volume and/or the variation thereof.

15. Process according to claim 14, **characterized in that** a first section (33) is given by the reconstruction of images of the response of pixels of the first column of all the planar detectors and **in that** acquisition takes place at the same time of the same number of sections as there are columns, the section height being defined by the dimension of the elementary pixel.

16. Process according to claim 15, **characterized in that** the section height is increased during acquisition by summating the response of several pixels on a line.
